# FASCICULE DE BREVET EUROPEEN

(11) **EP 1 620 390 B1**
(45) Date de publication et mention de la délivrance du brevet: **01.10.2008**
(21) Numéro de dépôt: 04742685.3
(22) Date de dépôt: 07.05.2004
(51) Int. Cl.: C07C 227/16, C07C 227/30, C07B 55/00, C07B 57/00

(54) **PROCEDE DE SYNTHESE DE LA 4-HYDROXYISOLEUCINE ET DE SES DERIVES**
VERFAHREN ZUR HERSTELLUNG VON 4-HYDROXYISOLEUCIN UND DESSEN DERIVATE
METHOD FOR THE SYNTHESIS OF 4-HYDROXYISOLEUCINE AND THE DERIVATIVES THEREOF

(30) Priorité: 07.05.2003 FR 0305568
(43) Date de publication de la demande: 01.02.2006
(73) Titulaire: CENTRE NATIONAL DE LA RECHERCHE SCIENTIFIQUE (CNRS), 75794 Paris Cedex 16 (FR); Université Louis Pasteur, 67070 Strasbourg Cedex (FR)
(72) Inventeur: MIOSKOWSKI, Charles, F-67200 Strasbourg (FR); CATALA, Cédric, F-67100 Strasbourg (FR); WAGNER, Alain, F-67000 Strasbourg (FR)
(74) Mandataire: Peaucelle, Chantal
(86) Numéro de dépôt international: PCT/FR2004/001128
(87) Numéro de publication internationale: WO 2004/099120

(56) Documents cités:
- EP-A- 0 623 580
- WO-A-01/72688
- BROCA C ET AL: "4-HYDROXYISOLEUCINE: EFFECTS OF SYNTHETIC AND NATURAL ANALOGUES ON INSULIN SECRETION" EUROPEAN JOURNAL OF PHARMACOLOGY, AMSTERDAM, NL, vol. 390, no. 3, mars 2000 (2000-03), pages 339-345, XP000908995 ISSN: 0014-2999
- INGHARDT T ET AL: "ORGANOALUMINIUM INDUCED RING-OPENING OF EPOXYPYRANOSIDES IV. SYNTHESIS AND STRUCTURE OF GAMMA- HYDROXY-ISOLEUCINE STEREOISOMERS AND THEIR CORRESPONDING LACTONES" TETRAHEDRON, ELSEVIER SCIENCE PUBLISHERS, AMSTERDAM, NL, vol. 47, no. 32, 5 août 1991 (1991-08-05), pages 6469-6482, XP000611434 ISSN: 0040-4020
- CHEMICAL ABSTRACTS, vol. 85, no. 9, 1976, Columbus, Ohio, US; abstract no.: 63319m, HASAN MASHOODA ET AL.: "The four diastereomeric lactones of gamma-hydroxyisoleucine" page 592 colonne 2 XP002266040 & JUSTUS LIEBIGS ANN. CHEM., vol. 4, 1976, pages 781-787,

## Description

L'invention a pour objet un procédé de préparation de la 4-hydroxyisoleucine et de ses dérivés, ce terme couvrant les analogues pouvant être obtenus par le procédé de l'invention. Elle vise en particulier la préparation de la (2S, 3R, 4S)-4-hydroxyisoleucine (4-OH-iLeu en abrégé) et de ses dérivés.
La 4-OH-iLeu est un produit naturel isolé de la graine de fénugrec, qui répond à la formule A :

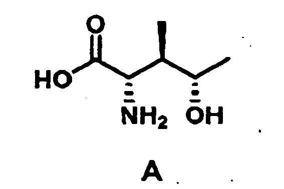

Ce produit est actif en particulier contre le diabète de type II, mais les quantités que l'on peut obtenir par extraction sont insuffisantes pour suppléer aux besoins des populations atteintes par ce type de diabète.

Les documents WO 01/72688 et EP 0 623 580 décrivent la synthèse de la (2S, 3R, 4S)-4-hydroxyisoleucine par réduction diastéréo et énantiosélective utilisant une enzyme.

EUR. J. OF PHARM., vol.390, n°3, mars 2000, pages 339-345 décrit l'effet insulinotropique supérieur de la 4-hydroxyisoleucine par rapport à celui de ses analogues.

TETRAHEDRON, vol.47, n°32, 5 août 1991, pages 6469-6482 décrit la préparation d'un diastéréoisomère de la gamma hydroxyisoleucine par réduction d'un dérivé aide.

CHEMICAL ABSTRACTS, vol.85, n°9, 1976, abstract n°63319m décrit les 4 diastéréoisomères lactoniques de la gamma-hydroxyleucine.

Si l'intérêt d'une obtention par voie de synthèse s'impose, il se heurte toutefois au problème du coût de réalisation.

Les recherches des inventeurs ont porté précisément sur la mise au point d'un procédé comportant un nombre réduit d'étapes de manière à le rendre exploitable à l'échelle industrielle.

L'invention vise donc un procédé de synthèse des deux isomères au niveau de la fonction OH, seuls ou en mélanges, des acides aminés α, ou de leurs dérivés,de formule générale B : 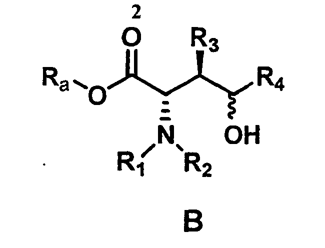 dans laquelle
- la liaison C-O du carbone en position 4 (représentée par « 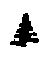 » symbolise l'un ou l'autre des isomères III ou IV, ou leurs mélanges,
- R₁ et R₂ représentent :
   - un atome d'hydrogène,ou
   - l'un de R₁ ou R₂ représente un atome d'hydrogène et l'autre substituant est un radical Rₐ, un groupe acyle -CORₐ, notamment acétyle, ou encore un groupe fonctionnel -COORₐ,-SO₂Rₐ,-N (Rₐ, R_{b}), Rₐ et R_{b}, identiques ou différents,étant un radical alkyle linéaire ou ramifié en C1-C12, le cas échéant substitué, un groupe aryle à un ou plusieurs cycles aromatiques et hétérocycles, comportant 5 à 8C, le cas échéant substitué, ou aralkyle, le substituant alkyle et le groupe aryle étant comme défini ci-dessus, ou
   - R₁ et R₂ représentent tous deux un substituant tel que défini ci-dessus,
- R₃ représente un atome d'hydrogène ou Rₐ, et
- R₄ présente les significations de Rₐ,
caractérisé en ce qu'il comprend :
- l'isomérisation d'un composé de formule I : 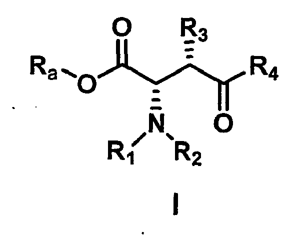
dans laquelle R₁, R₂, Rₐ, R₃ et R₄ sont tels que définis ci-dessus,
de manière à obtenir un composé de formule II : 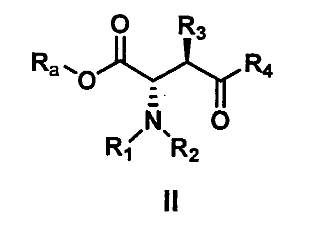
- la réduction de sa fonction carbonyle ce qui conduit, selon le système catalytique utilisé et le composé de formule I mis en oeuvre, à l'un des isomères de formule générale III ou IV, : 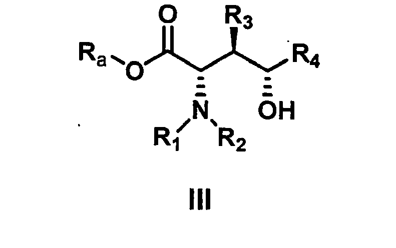 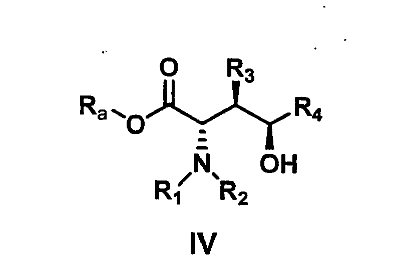
ou à leur mélange de formule B
les substituants Rₐ et R₁ à R₄ étant tels que définis ci-dessus.

La transformation du diastéréoisomère I en diastéréoisomère II, tous deux énantiomériquement purs, est une isomérisation dynamique qui permet, par cristallisation spécifique du composé II, de déplacer complètement l'équilibre de I vers II et d'isoler le composé II pur, si souhaité.

Dans un mode de réalisation de l'invention, le procédé de synthèse est caractérisé en ce que l'étape d'isomérisation est suivie d'une étape de déprotection totale ou partielle de la fonction amine, conduisant à la formation du composé XXIII 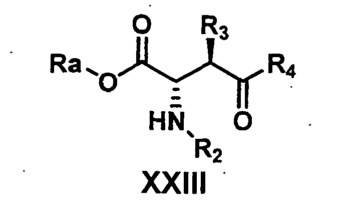 puis d'une étape de réduction de la fonction carbonyle, de manière à conduire, comme composé majoritaire ou exclusif, à la lactone de formule VII 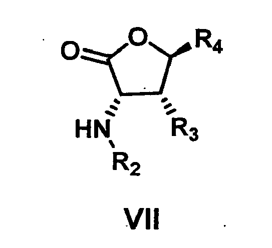 dont l'ouverture conduit majoritairement au composé de formule C

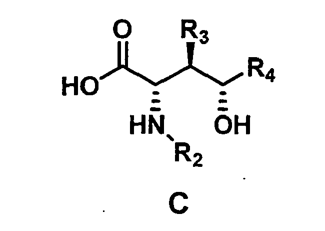

Le composé C est purifié par recristallisation, si nécessaire.

Dans un autre mode de réalisation de l'invention, le procédé de synthèse est caractérisé en ce que l'isomère III obtenu par isomérisation du composé I et réduction du composé II est soumis à une hydrolyse de sa fonction ester pour conduire au composé de formule V : 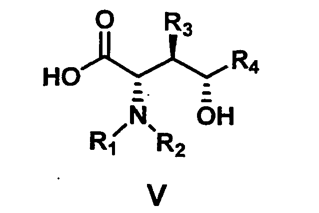 dont au moins l'un des groupements protecteurs de la fonction azote est éliminé, si souhaité, pour conduire au composé de formule générale C :

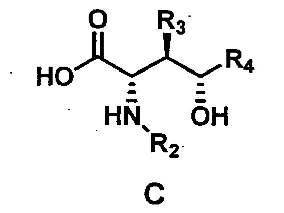

Dans un autre mode de réalisation, le groupe protecteur R1 de III peut être éliminé selon les méthodes classiques, ce qui conduit au composé de formule VI : 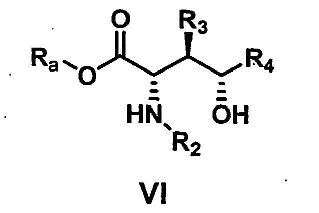 dont la fonction ester est hydrolysée pour conduire au composé C.

En variante, le composé de formule VI est cyclisé en lactone de formule VII : 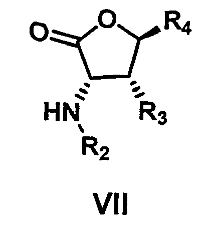 dont l'ouverture conduit ensuite au composé de formule C ci-dessus.

L'isomère de formule IV peut être soumis aux mêmes séquences, conduisant à un produit de formule générale D :

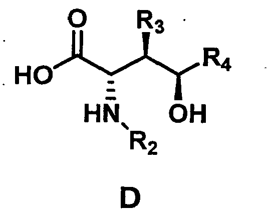

Dans encore un autre mode de réalisation de l'invention, l'isomère II ou l'isomère III conduit par cyclisation à une lactone de formule VIII ou IX : 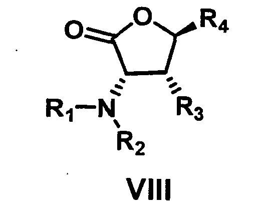 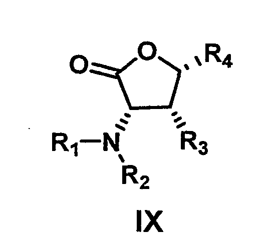 qui peuvent être séparées, par exemple par chromatographie ou cristallisation.

La lactone VIII peut être transformée en lactone VII par déprotection totale ou partielle de la fonction amine et la lactone IX en lactone X :

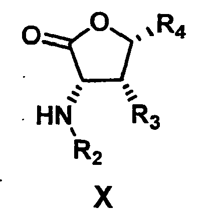

Les lactones VII et X sont respectivement ensuite hydrolysées pour conduire aux composés de formules générales C et D ci-dessus.

L'étape d'isomérisation est réalisée, avec ou sans solvant, en présence d'une base, en se plaçant dans des conditions de précipitation du composé II, ce qui déplace l'équilibre vers ce composé.

Des solvants appropriés comprennent l'éthanol, le benzène, le toluène, et de préférence des solvants aprotiques, tels que l'hexane, le tétrahydrofurane, ou le tert-butylméthyléther.

La base est, par exemple, un phosphazène, ou une base aminée, comme la triéthylamine, la DBN (1,4-Diazabicyclo[4.3.0]non-5-ene) ou la DBU (1,8-Diazabicyclo[5.4.0]undec-7-ène).

On peut également utiliser des systèmes organo-métalliques, des bases supportées sur des polymères, par exemple des amines greffées sur polystyrène, des hydroxydes d'ammonium supportés ou des aluminosilicates, en variante, un sel de métal alcalin, comme la potasse ou le carbonate de potassium.

Après filtration du composé précipité II, on opère la recristallisation de l'isomère II dans un solvant approprié comme le méthanol, l'hexane ou préférentiellement l'éthanol, à des températures allant de -60°C à 30°C.

Comme indiqué plus haut, l'isomère II peut être soumis à une étape de déprotection totale ou partielle de la fonction amine. Cette déprotection est réalisée de manière classique. Dans le cas où R₁ représente un groupement protecteur oxydable, comme le p-méthoxyphényle, on a recours, par exemple, à du nitrate de cérium et d'ammonium, ou à la voie électrochimique, ou on utilise comme réactifs des persulfates (ammonium, sodium, potassium...), des perborates, du dioxyde de manganèse, du permanganate de potassium, H₂O₂, FeCl₃, ou les combinaisons cérium et oxydant.

Cette étape n' est suivie d'aucune purification et une simple extraction permet d'enchaîner sur la réduction en milieu aqueux de la fonction carbonyle par exemple avec du borohydrure de potassium en milieu aqueux accompagné de CeCl₃ en quantité catalytique. Cette étape conduit au mélange des deux diastéréoisomères possibles, le composé VII décrit dans la littérature étant majoritaire, en passant par l'intermédiaire ouvert (non isolé) de formule VI.

En variante, l'isomère II récupéré à l'issue de l'étape d'isomérisation est soumis à une étape de réduction de la fonction carbonyle.

En fonction des conditions opératoires utilisées, la formation de l'un des composés III, IV, VIII ou IX peut être favorisée.

Le produit déprotégé est soumis à une étape de réduction en milieu aqueux de la fonction carbonyle, ce qui conduit de manière majoritaire au composé VII.

La réduction s'effectue par exemple dans un solvant de type éthanol, auquel on ajoute un catalyseur de réduction, si nécessaire un modificateur de réactivité et l'isomère II, puis on purge à l'hydrogène. Le milieu réactionnel est ensuite agité sous une pression d'hydrogène allant de 1 bar à 50 bars à température ambiante.

Pour favoriser la formation des composés III et IV, la réduction est réalisée sous une pression d'hydrogène de l'ordre de 50 bars.

Pour favoriser la formation des composés VIII et IX, la réduction est réalisée sous une pression d'hydrogène de l'ordre de l'atmosphère.

Un exemple de l'invention consiste à favoriser l'obtention majoritaire d'un des deux diastéréoisomères VIII ou IX en variant la nature du catalyseur et de l'additif utilisé.

En particulier, la réduction, sous 1 atmosphère, de II en présence de ruthénium black conduit préférentiellement à la formation du composé IX, et, la réduction de II en présence de nickel de Raney conduit préférentiellement à la formation du composé VIII.

Un exemple d'additif utilisé est le DABCO (1,4-Diazabicyclo[2.2.2]octane) qui permet d'inverser la sélectivité du nickel de Raney.

Ainsi, une réduction effectuée par exemple, sous 1 atmosphère d'hydrogène, dans un solvant de type éthanol, auquel on ajoute du nickel de Raney en suspension dans l'éthanol, du DABCO et l'isomère II, conduit préférentiellement à la formation du composé IX.

Quelle que soit la séquence, l'hydrolyse de la fonction ester des produits III ou IV est effectuée par traitement en milieu alcalin, avantageusement avec LiOH, ou dans un solvant alcoolique aqueux en opérant selon des méthodes connues en soi.

Les lactones de formules VIII ou IX sont également hydrolysées par exemple en utilisant LiOH dans THF.

L'élimination du groupement R₁, quelle que soit la séquence, est avantageusement effectuée selon les méthodes classiques par exemple, l'élimination du groupe para-méthoxyphényle est obtenue par traitement avec un nitrate de cérium et d'ammonium (CAN), ou par voie électrochimique.

Conformément à l'invention, le composé de formule I est avantageusement obtenu par condensation d'une cétone de formule XI : 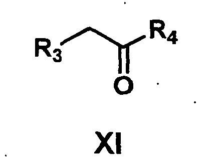 avec une imine de formule XII : 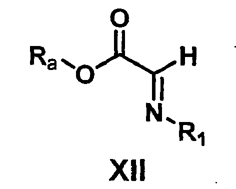 en présence d'un catalyseur chiral de formule générale XIII, qui peut être de configuration R ou S au niveau du carbone en position 2 :

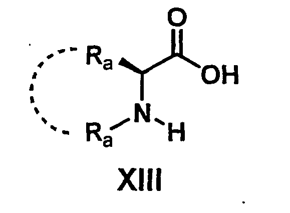

Les deux substituants Rₐ, identiques ou différents, sont tels que définis ci-dessus, et peuvent former en outre un cycle, notamment de 5 à 8 éléments.

D'autres catalyseurs de condensation énantiospécifiques peuvent également être utilisés.

On réalise avantageusement l'étape de condensation dans un solvant polaire ou ionique, en présence d'un catalyseur recyclable. En fonction de la stéréochimie du catalyseur, on obtient l'un ou l'autre des composés de formule générale I, ou son image XIV :

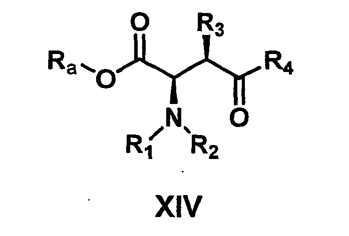

Le composé XIV peut subir les mêmes étapes que le composé de formule I.

Après la condensation, R₂ dans le composé de formule I représente un atome d'hydrogène. Un groupement R₂ différent de l'hydrogène peut être introduit, en opérant selon les méthodes classiques, si souhaité. Ainsi, pour introduire un substituant alkyle, carbamyle, sulfonyle, acyle, notamment acétyle, on utilise, par exemple, un agent d'alkylation, de carbamylation, de sulfonylation ou d'acylation approprié, avantageusement de l'anhydride acétique pour synthétiser les dérivés acétylés.

Des solvants appropriés comprennent le diméthylsulfoxyde (DMSO), l'éthanol (EtOH), ou encore le diméthylformamide (DMF). Des catalyseurs particulièrement satisfaisants comprennent la L-proline ou les dérivés de la trans-hydroxy proline.

Les imines de formule XII sont obtenues de manière avantageuse par condensation d'un glyoxalate XV 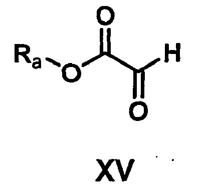 avec une amine R₁NH₂.

L'invention vise tout particulièrement l'obtention de la 4-hydroxy-isoleucine de formule A ci-dessus, selon un procédé comprenant :
- la condensation du composé de formule XVI 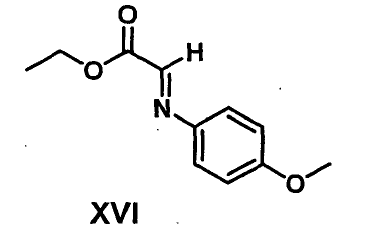 sur la 2-butanone, en présence de L-proline, ce qui conduit, de manière majoritaire, au composé de formule XVII, 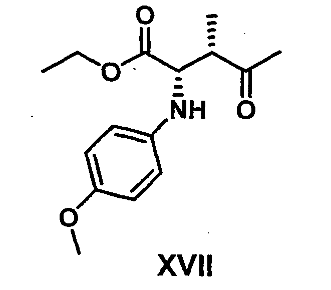
- suivie de son isomérisation/cristallisation par traitement de XVII, avec de la DBU ou de la DBN, ce qui conduit au composé XVIII : 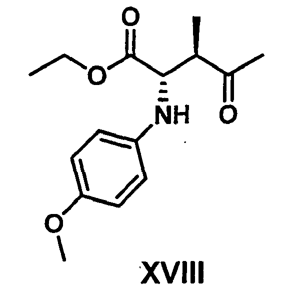
- l'élimination du groupement protecteur para-méthoxy phényle par traitement avec un persulfate,
- la réduction du composé déprotégé avec KBH₄ /CeCl₃ dans de l'éthanol ou dans l'eau, ce qui conduit à la lactone XX: 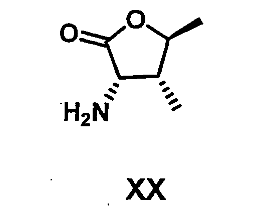
qui par hydrolyse dans LiOH/THF conduit majoritairement au composé A.Ce composé est isolé et cristallisé.

Dans un autre mode de synthèse de la 4-hydroxy-isoleucine, le composé XVIII est réduit avant d'être déprotégé.

L'invention vise également, en tant que produits nouveaux, les composés intermédiaires des étapes opératoires ci-dessus.

L'invention vise ainsi en particulier les composés répondant à la formule générale II. Un produit plus spécialement préféré correspond au composé XVIII.

Elle vise également les composés intermédiaires de formule générale XXIII. Un composé particulièrement préféré répondant à cette formule correspond au composé XXIV.

L'invention vise encore, en tant que tels, les produits intermédiaires de formule générale VIII. Elle vise spécialement le produit XIX.

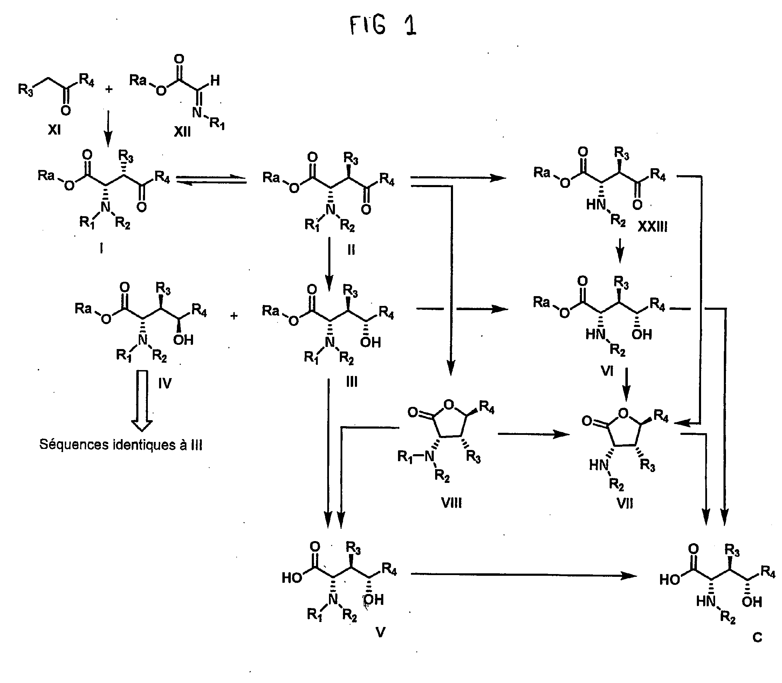

Afin d'illustrer l'invention, on décrit ci-après des exemples de réalisation des étapes mentionnées ci-dessus. Un schéma général des réactions pouvant être mises en jeu est donné sur la figure unique.

### Exemple I : Obtention des imines.

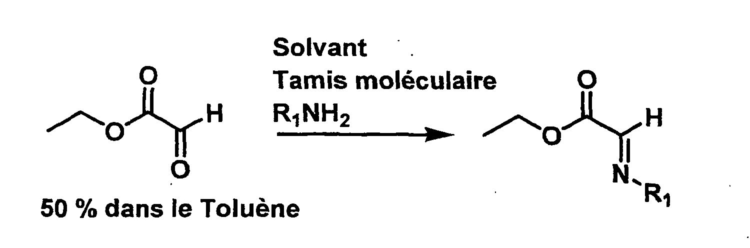

### Mode opératoire général

Dans un ballon bicol, flambé sous argon avec 6g de tamis moléculaire, 1 g de glyoxalate d'éthyle (V = 2 mL) est ajouté dans 20 mL de solvant anhydre (toluène ou dichlorométhane selon la stabilité de l'imine formée). Puis, 1 équivalent d'amine est additionné et le mélange est agité vigoureusement pendant 2 heures à température ambiante.

Le brut réactionnel est filtré sur Célite® puis évaporé sous pression réduite. Il est utilisé tel quel sans purification dans l'étape suivante de condensation.

| **Entrée** | **Imine formée** | **Rdt** | **Entrée** | **Imine formée** | **Rdt** |
|---|---|---|---|---|---|
| 1 | 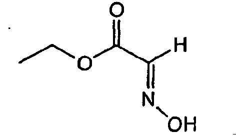 | 90 % | 2 | 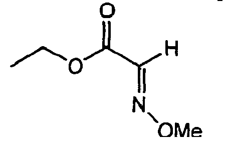 | 40 % |
| 3 | 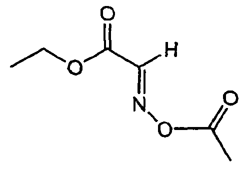 | 70 % | 4 | 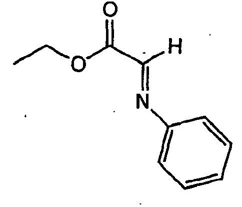 | 95 % |
| 5 | 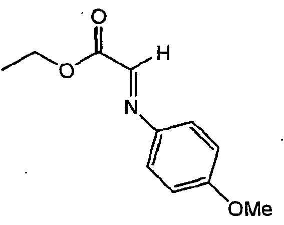 | 98 % | 6 | 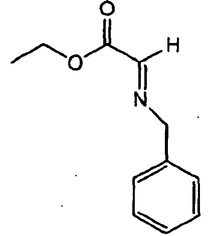 | 86 % |
| 7 | 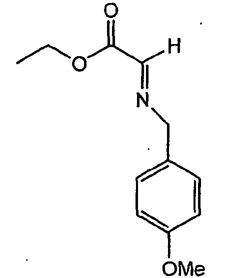 | 95 % | 8 | 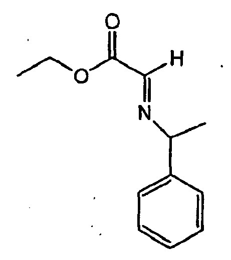 | 97 % |
| 10 | 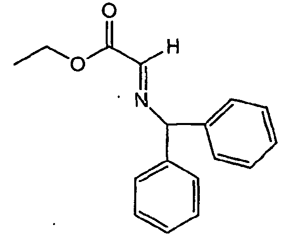 | 95 % | 11 | 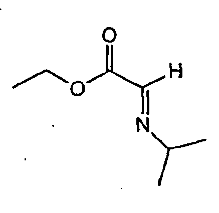 | 90 % |
| 12 | 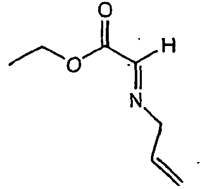 | 98 % | 13 | 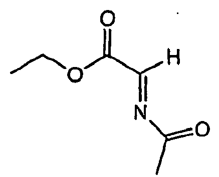 | 55 % |
| 14 | 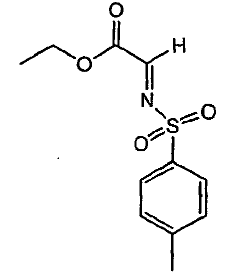 | 80 % | | | |

### Exemple II : Obtention d'un mélange d'isomères E, F et G selon le schéma suivant :

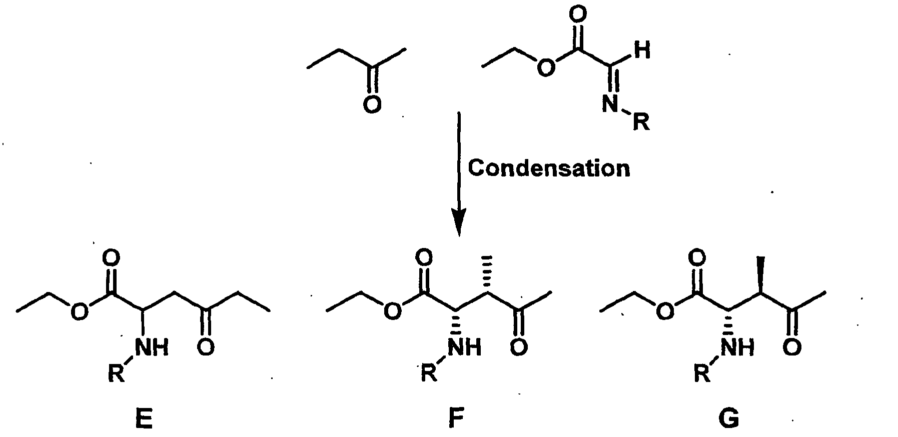

avec R = - PMP (para-méthoxyphényl)

### Mode opératoire A

Dans un ballon bicol, on place 2,8 ml de solvant, 2 ml de butanone et 0,35 éq. de L-proline, puis l'imine (1 mmol) est ajoutée. Le milieu réactionnel est agité pendant 2h, puis dilué dans 10 ml d'acétate d'éthyle et 10 ml de solution de tampon phosphate à pH = 7,4. La phase organique est recueillie, séchée sur MgSO₄, puis filtrée sur Célite®. Le brut réactionnel est évaporé et chromatographié sur colonne de silice.

On a résumé dans le tableau 1 ci-après les conditions opératoires et les résultats obtenus.

**Tableau 1**

| **N° exp.** | **Solvant (conc.)** | **catalyseur** | **Temps de réaction** | **Rendement (F)** | **Excès énantiomérique (F)** | **Rapport G/F/E** |
|---|---|---|---|---|---|---|
| 1 | DMSO (0.125 M) | L proline | 2h15 | 75 % | 99/1 | 0/90/10 |
| 2 | DMSO (0.125 M) | L Proline | 2h53 | 71 % | 100/0 | 0/88/12 |
| 3 | DMSO (0.4 M) | L proline | 3h34 | 71 % | 100/0 | 0/88/12 |
| 4 | DMSO (0.125 M) | DL proline | 3h05 | 73 % | 50/50 | 0/88/12 |
| 5 | DMSO (0.125 M) | T hydroxy proline | 23h30 | 73 % | 100/0 | 0/88/12 |
| 6 | EtOH (0.125 M) | L proline | 3h00 | 70 % | 96/4 | 0/91/8 |
| 7 | DMF (0.125 M) | L proline | 3h45 | 73 % | 100/0 | 0/90/10 |
| 8 | 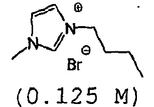 | L proline | 3h30 | 61 % | 100/0 | 0/96/4 |
| 9 | 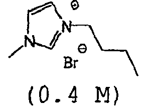 | L proline | 1h20 | 61 % | - | 0/95/5 |
| 10 | 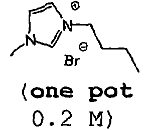 | L proline | 19h00 | 53 % | | 0/91/8 |
| 11 | 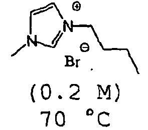 | T hydroxy proline | 1h20 | 44 % | - | 0/95/5 |

### Autres exemples de condensation

**Tableau 2**

| **Produit** | **G/F/E** | **Rendement GC** |
|---|---|---|
| 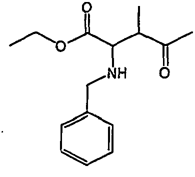 | 70/30/0 | 62 % |
| 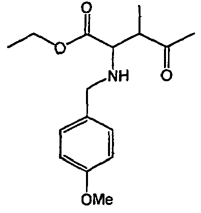 | 60/40/0 | 50 % |
| 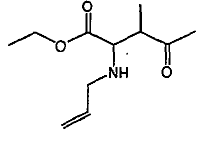 | 70/30/0 | 77 % |
| 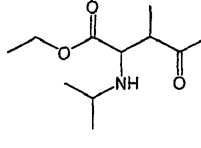 | 70/30/0 | 46 % |
| 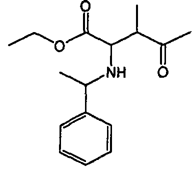 | Exception car 4 dia en même proportions | 20 % |
| 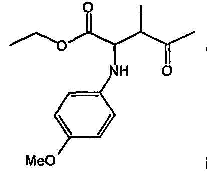 | 0/95/5 | 88 % |
| 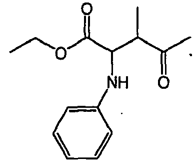 | 0/88/12 | 78 % |
| 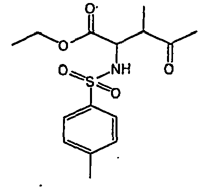 | 10/70/20 | 65 % |

### Exemple III : Isomérisation de l'isomère F.

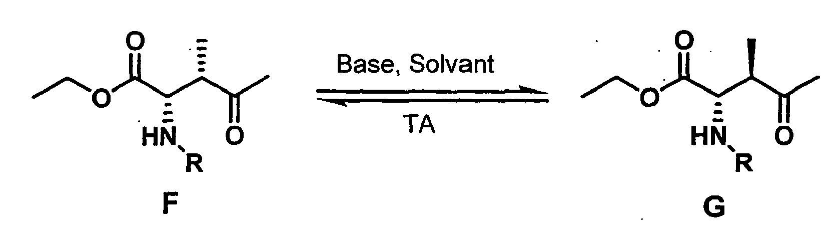

**Tableau 3**

| R= -PMP | | | |
|---|---|---|---|
| Solvant | Base | F | G |
| EtOH | Et₃N | 40 | 60 |
| Toluène | DBN | 40 | 60 |
| TBME | DBN | 40 | 60 |
| Aucun | DBN | 20 | 80 |
| TBME (méthode par évaporation) | DBN | 5 | 95 |

### Mode opératoire B

### Exemple IV : Réduction de la fonction carbonyle et lactonisation.

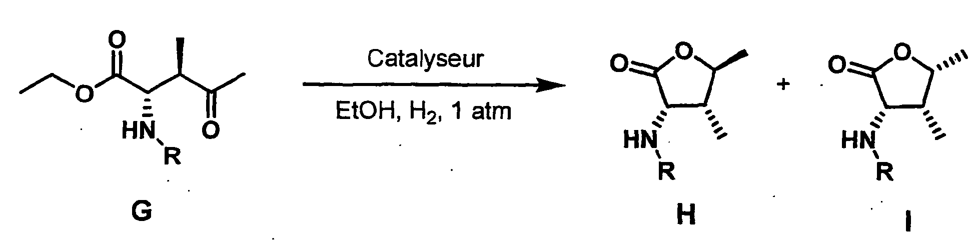

Les résultats obtenus sont donnés dans le Tableau 4.

**Tableau 4 (R= -PMP)**

| Catalyseur | H | I |
|---|---|---|
| NiR/ H₂O | 60 | 40 |
| NiR/DABCO | 30 | 70 |
| NiR/ B(iPrO)₃ | 70 | 30 |
| Ni Sponge | 50 | 50 |
| Ru Black | 25 | 75 |

### Mode opératoire.

Dans un ballon monocol de 5 mL sont introduits l'éthanol (1mL), la solution de Nickel de Raney dans l'éthanol (100 µL) et G (20 mg, 1eq, 0,71.10⁻⁴ mol). L'ensemble est porté à 0°C puis purgé à l'hydrogène.

Le milieu est agité sous pression d'hydrogène (1atm) pendant 24 heures à température ambiante.

Le brut réactionnel est purifié par chromatographie sur colonne de silice et les composés H et I isolés avec un rendement de 90%. Les proportions de chaque diastéréoisomères sont définies par RMN ¹H.

### Réduction de la fonction carbonyle sans lactonisation

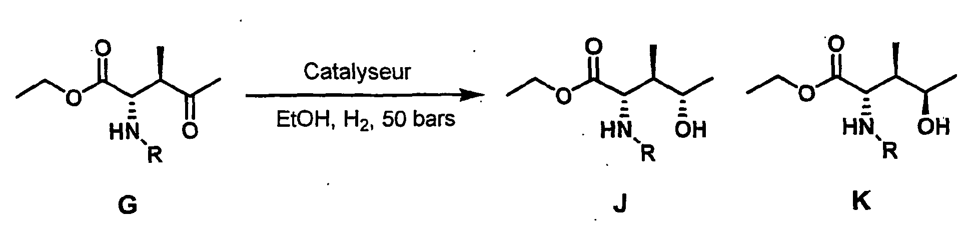

**Tableau 5**

| Catalyseur | J | K |
|---|---|---|
| NiR | 60 | 40 |
| Pt/C | 60 | 40 |

### Mode opératoire.

Dans un autoclave de 100 mL sont introduits l'éthanol (3mL), la solution de Nickel de Raney dans l'éthanol (300 µL) et G (60 mg, 1eq, 2,1.10⁻³ mol). L'ensemble est purgé trois fois à l'hydrogène.

Le milieu est agité sous une pression d'hydrogène de 50 bars pendant 24 heures à température ambiante.

Le brut réactionnel est purifié par chromatographie sur colonne de silice et les composés J et K isolés avec un rendement supérieur à 90%. Les proportions de chaque diastéréoisomère sont définies par RMN ¹H.

### Exemple V : Elimination du groupement R₁

- selon le schéma suivant :

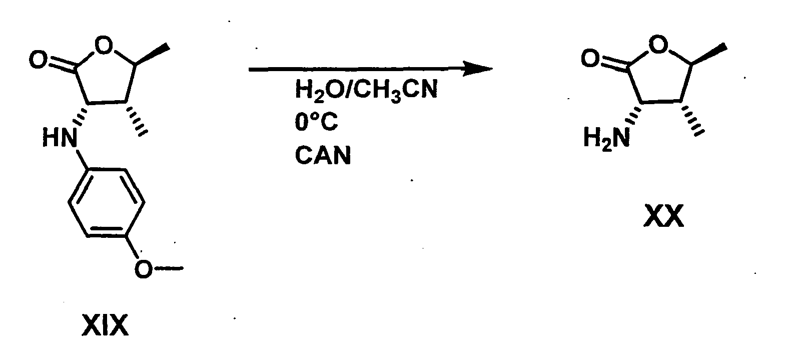

### Mode opératoire :

- dans un ballon bicol à 0°C, on place la lactone de départ (1 mmol) dans CH₃CN (4,9 mL) et on additionne CAN (30 g) dans 1 mL d'eau. On laisse agiter 40 min, puis on ajoute 10 mL d'eau avec 20 mL de dichlorométhane. La phase aqueuse est extraite à trois reprises avec 10 ml de dichlorométhane, puis est basifié par ajout de carbonate de sodium jusqu'à pH 9.
- la phase aqueuse basique est alors extraite sept fois avec du dichlorométhane. Les phases organiques sont rassemblées et séchées sur MgSO₄, puis évaporées.
- le produit désiré est obtenu avec un rendement de 38%.

En variante, la déprotection est effectuée par voie électrochimique en utilisant une électrode de travail en platine, une contre électrode de platine et une électrode de référence Ag/AgCl. On se place à 0,7 V pendant 5h. Le traitement est identique à celui de la méthode classique. La lactone désirée est obtenue avec un rendement de 21%

On applique les mêmes méthodes pour obtenir le composé XXII à partir du composé XXI.

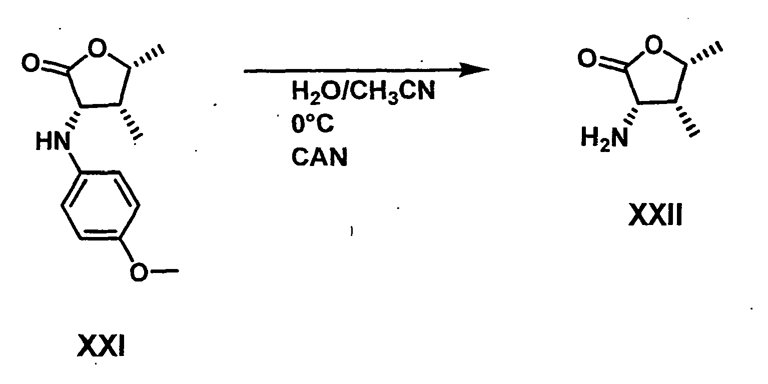

### Hydrolyse et purification

Le mélange de lactones (90/10) est dissous dans 96 mL d'eau (0,3M) et de l'hydroxyde de lithium (1,1g, 43,3 mmol, 1,5 eq) est additionné à température ambiante. Après deux heures d'agitation, le milieu réactionnel est acidifié à l'aide d'acide acétique (43,3 mmol, 2,4 mL) et l'ensemble est ensuite porté sous vide afin d'éliminer toute trace d'eau, de solvant et d'acide.

La gomme obtenue est dissoute dans l'éthanol et 1,56g de la (2S, 3R, 4S)-4-hydroxyisoleucine pure sont sélectivement recristallisés (80% de rendement).

### Analyses

Les analyses GC/MS sont toutes faites sur le même type de matériel.
*GC*/*MS (Shimadzu GCMS-QP5050A)*
*Colonne SGE CAPILLARY Silice 25m x 0,22mm BPX5 0,25*

### Programme

Interface : 260°C
Colonne : 80°C
Détecteur : 320°C
2 min à 80°C puis montée en température de 10°C /min

Les analyses HPLC sont toutes faites sur le même type de matériel.

*HPLC (Gynkotek Gina 50)* et colonne *ZORBAX SIL 4.6 MM ID x 25cm*
Eluant : Hexane/Ethanol 95/05
Débit : 6 mL/min

### Composé XX

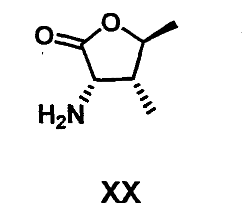

**RMN ¹H (CDCl₃, 200 MHz)** δ **(ppm) :** 1.10 (d), 1.40 (d), 1.82 (s), 2.30 (m), 3.80 (d), 4.32 (m)
**SM(IC) m/z :** [M+H]⁺ = 130
**GC/MS t_{R} =** 6.22 min

Composé XXII 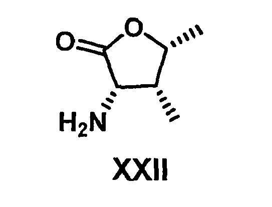 **RMN ¹H (CDCl₃, 300 MHz)** δ **(ppm)** : 0.91 (d), 1. 36 (d), 1. 82 (s), 2.59 (m), 3.85 (d), 4.53 (m)
**SM(IC) m/z** : [M+H]⁺ = 130
**GC/MS t_{R}** = 6. 69 min
Composé XIX 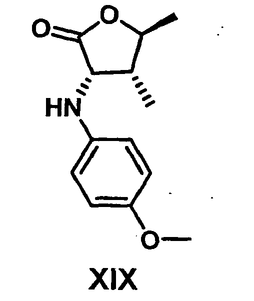 **RMN ¹H (CDCl₃, 300 MHz)** δ **(ppm) :** 0.99 (d), 1. 48 (d), 2.65 (m), 3.74 (s), 3.98 (s), 4.24 (m), 4.40 (q), 6.71 (2*d)
**RMN ¹³C (CDCl₃, 75 MHz)** δ **(ppm)** : 13.36 ; 20.31 ; 40.25 ; 55.60 ; 55.95 ; 81.82 ; 114.15 ; 114.85 ; 128.21 ; 140.75 ; 152.72 ; 175.78
**SM(IC) m/z :** [M+H]⁺ = 236
**HPLC t_{R} =** 5.053 min

Composé XXI 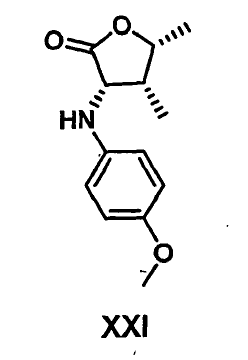 **RMN ¹H (CDCl₃, 300 MHz)** δ **(ppm)** : 0.82 (d), 1.39 (d), 2.89 (m), 3.74 (s), 4.13 (s), 4.19 (m), 4.68 (m), 6.71 (2*d)
**RMN ¹³C (CDCl₃, 75 MHz)** δ **(ppm)** : 6.9 ; 15.31 ; 39.33 ; 55.60 ; 59.39 ; 76.86 ; 113.99 ; 114.88 ; 140.58 ; 152.65 ; 175.84
**SM(IC) m/z** : [M+H]⁺ = 236
**HPLC t_{R} =** 5.382 min

Composé XVII 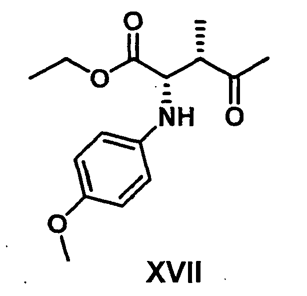 **RMN ¹H (CDCl₃, 200 MHz)** δ **(ppm)** : 1.21 (t), 1.23 (d), 2.21 (s), 3.01 (m), 3.72 (s), 3.90 (s), 4.15 (q), 4.35 (d), 6.70 (2*d)
**RMN¹³C : (CDCl₃, 200 MHz)** δ **(ppm)** : 209.58 ; 173.20 ; 153.50 ; 141.19 ; 116.18 ; 115.20 ;61.72 ; 59.98 ; 56.05 ; 49.62 ; 28.89 ; 14.54 ; 12.64
**SM(IC) m/z** : [M+H]⁺ = 280
**GC/MS t_{R}** = 17.33 min

Composé XVIII

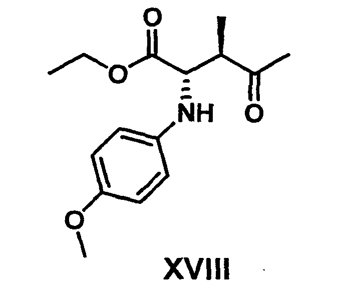

### Chiralité

Colonne HPLC : Chiralpak AS
Eluant : 2Hexane/EtOH 90/10
Débit : 1mL/min
UV : 220 nm
Température : 30°C
**Temps de rétention : 6,67 min**
α_{D} : -35,5 **(CH₂ Cl₂** C=1, 0)
**mp :** 98,8-99,1 °C
**RMN¹H : (CDCl₃, 300 MHz)** δ **(ppm) :** 1.19 (d), 1. 22 (t), 2.23 (s), 3.02 (m), 3.73 (s), 4.15 (q, d, s), **6.74 (2*d)**
**RMN ¹³C (CDCl₃, 50 MHz)** δ **(ppm) :** 12.85 ; 14.00 ; 28.47 ; 49.19 ; 55.48 ; 60.30 ; 61.09 ; 114.67 ; 115.60 ; 140.60 ; 152.90 ; 172.38 ; 209.35
**SM(IC) m/z :** [M+H]⁺ = 280
**GC/MS t_{R}** = 17.27 min
**IR : ν(NH) = 3357 cm⁻¹**
**v(CO) = 1733 cm⁻¹ et 1715 cm⁻¹**

Composé XVI 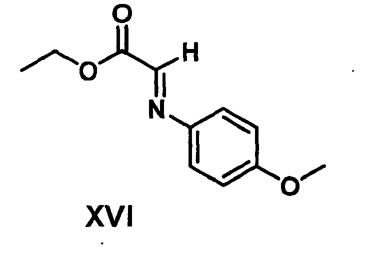 **RMN ¹H (CDCl₃, 300 MHz)** δ **(ppm)** : 1.38(d), 3.82 (s), 4.39 (q), 6.91 (d), 7.35 (d), 7.92 (s)
**RMN ¹³C (CDCl₃, 75 MHz)** δ **(ppm)** : 14.13 ; 55.20 ; 59.81 ; 118.28 ; 122.01 ; 142.1 ; 143.61 ; 157 ; 165.23
**SM(IC) m/z :** [M+H]⁺ = 208
**GC/MS t_{R}** = 8.47 min
(montée de 25 °C/min)

Composé XXIV

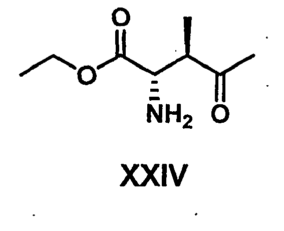

### Gomme

**RMN¹H : (CDCl₃, 300 MHz)** δ **(ppm)** : 0.94 (d), 1. 03 (t), 1.50 (s), 1.98 (s), 2.71 (m), 3.33 (d), 3.94 (m)
**RMN¹³C : (CDCl₃, 50 MHz)** δ **(ppm) :** 12.69 ; 13.50 ; 28.22 ; 49.78 ; 56.26 ; 60.30 ; 173.76 ; 209.45
α**_{D} :-52,9 (CH₂Cl₂ C=1,0**)
**SM (IC) m/z :** [M+H⁺] = 174
IR : ν(CO) = 1734 cm⁻¹ et 1716 cm⁻¹

## Revendications

1. Procédé de synthèse des deux isomères, au niveau de la fonction OH, seuls ou en mélanges, des acides aminés α, ou de leurs dérivés, de formule générale B : 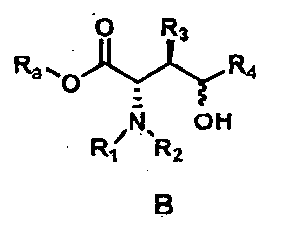 dans laquelle
- la liaison C-O du carbone en position 4 représentée par « 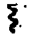 » symbolise l'un ou l'autre des isomères III ou IV, ou leurs mélanges,
- R₁ et R₂ représentent :
. un atome d'hydrogène,ou
. l'un de R₁ ou R₂ représente un atome d'hydrogène et l'autre substituant est un radical Rₐ, un groupe acyle -CORₐ, ou encore un groupe fonctionnel -COORₐ, -SO₂Rₐ, -N (Rₐ, R_{b}), Rₐ et R_{b}, identiques ou différents,étant un radical alkyle linéaire ou ramifié en C1-C12, le cas échéant substitué, un groupe aryle à un ou plusieurs cycles aromatiques et hétérocycles, comportant 5 à 8C, le cas échéant substitué, ou aralkyle, le substituant alkyle et le groupe aryle étant comme défini ci-dessus, ou
- R₁ et R₂ représentent tous deux un substituant tel que défini ci-dessus,
- R₃ représente un atome d'hydrogène ou Rₐ, et
- R₄ présente les significations de Rₐ,
**caractérisé en ce qu'**il comprend :
- l'isomérisation d'un composé de formule I : 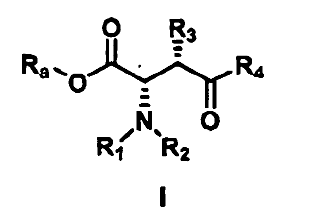
dans laquelle R₁, R₂, Rₐ, R₃ et R₄ sont tels que définis ci-dessus,
de manière à obtenir un composé de formule II : 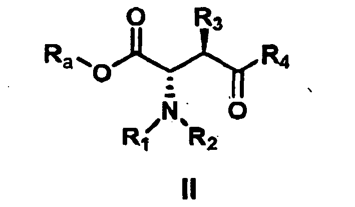
- la réduction de sa fonction carbonyle ce qui conduit, selon le système catalytique utilisé et le composé de formule I mis en oeuvre, à l'un des isomères de formule générale III ou IV, ou à leur mélange de formule B : 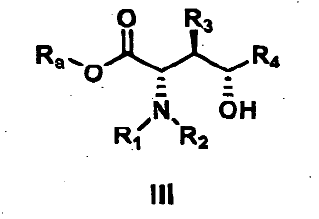 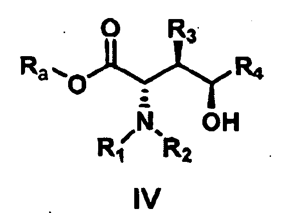
les substituants Rₐ et R₁ à R₄ étant tels que définis ci-dessus.

2. Procédé selon la revendication 1, **caractérisé en ce que** l'étape d'isomérisation est suivie d'une étape de déprotection totale ou partielle de la fonction amine, puis d'une étape de réduction de la fonction carbonyle, de manière à conduire, comme composé majoritaire ou exclusif, à la lactone de formule VII 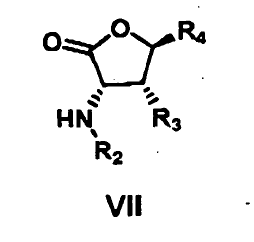 dont l'ouverture conduit à un mélange renfermant majoritairement le composé de formule C

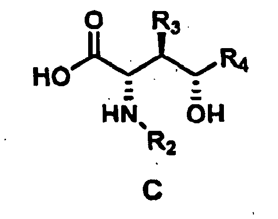

3. Procédé selon la revendication 1, **caractérisé en ce que** l'isomère III obtenu par isomérisation du composé I et réduction du composé II est soumis à une hydrolyse de sa fonction ester pour conduire au composé de formule V : 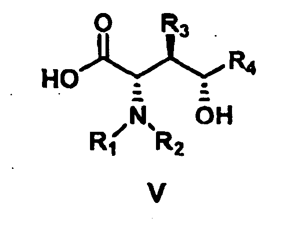 dont au moins l'un des groupements protecteurs de la fonction azote est éliminé, si souhaité, pour conduire au composé de formule générale C :

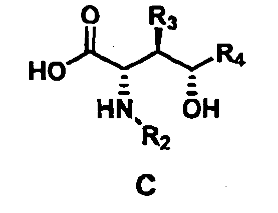

4. Procédé selon la revendication 3, **caractérisé en ce que** le groupe protecteur R1 du composé III est éliminé, ce qui conduit au composé de formule VI : 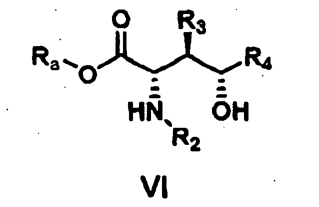 dont la fonction ester est hydrolysée pour conduire au composé C.

5. Procédé selon la revendication 3, **caractérisé en ce que** le composé de formule VI est cyclisé en lactone de formule VII : 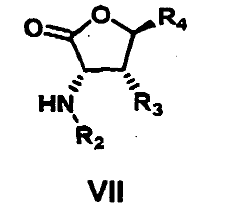 l'ouverture de cette lactone conduisant ensuite au composé de formule C, ou que l'isomère II est réduit, puis cyclisé en composé de formule VIII : 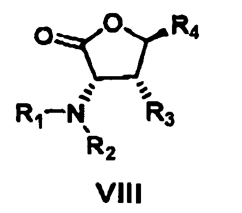 qui est déprotégé en composé de formule VII conduisant ensuite au composé dé formule C.

6. Procédé selon la revendication 1, **caractérisé en que** l'isomère de formule IV est soumis aux séquences selon les revendications 2 ou 3, ce qui conduit à un produit de formule générale D :

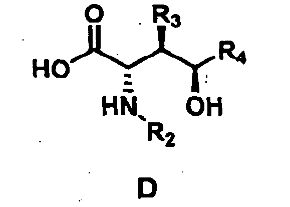

7. Procédé selon la revendication 1, **caractérisé en ce que** l'isomère II est réduit et cyclisé de manière à conduire à une lactone de formule VIII ou IX : 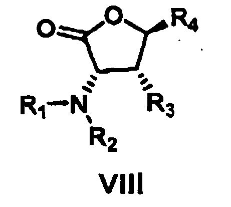 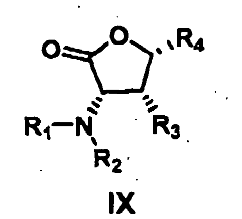 ces lactones pouvant être séparées les unes des autres, si souhaité.

8. Procédé selon la revendication 6, **caractérisé en ce que** la lactone VIII est transformée en lactone VII par déprotection totale ou partielle de la fonction amine et la lactone IX en lactone X : 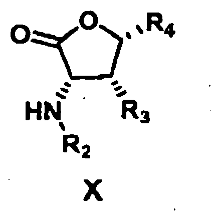 ces lactones VII et X étant ensuite, respectivement, hydrolysées pour conduire aux composés de formules générales C et D selon la revendication 2.

9. Procédé selon l'une quelconque des revendications 1 à 3, **caractérisé en ce que** l'étape d'isomérisation est réalisée avec ou sans solvant, en présence d'une base, en se plaçant dans des conditions de précipitation du composé II, ce qui déplace l'équilibre vers ce composé.

10. Procédé selon l'une quelconque des revendications 1 à 3, **caractérisé en ce que** l'isomère II est soumis à une étape de déprotection totale ou partielle de la fonction amine, cette étape étant réalisée dans le cas où R₁ représente un groupement protecteur oxydable, on a recours à du nitrate de cérium et d'ammonium, ou à la voie électrochimique, ou on utilise comme réactifs des persulfates, des perborates, du dioxyde de manganèse, du permanganate de potassium, H₂O₂, FeCl₃, ou les combinaisons cérium et oxydant.

11. Procédé selon la revendication 11, **caractérisé en ce que** les persulfates sont choisis parmi ceux d'ammonium, sodium ou potassium.

12. Procédé selon la revendication 10, **caractérisé en ce que** le produit déprotégé est soumis à une étape de réduction en milieu aqueux de la fonction carbonyle, ce qui conduit de manière majoritaire au composé VII.

13. Procédé selon la revendication 1, **caractérisé en ce que** l'isomère II récupéré à l'issue de l'étape d'isomérisation est soumis à une étape de réduction de la fonction carbonyle.

14. Procédé selon la revendication 13, **caractérisé en ce que** l'hydrolyse de la fonction ester des produits III ou IV est effectuée par traitement en milieu alcalin, ou dans un solvant alcoolique aqueux.

15. Procédé selon l'une quelconque des revendications 4,6 ou 7, **caractérisé en ce que** les lactones de formules VIII ou IX sont hydrolysées en utilisant LiOH dans THF.

16. Procédé selon l'une quelconque des revendications précédentes, **caractérisé en ce que** le composé de formule I est obtenu par condensation d'une cétone de formule XI : 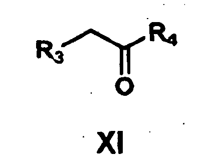 avec une imine de formule XII : 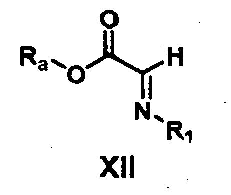 en présence d'un catalyseur chiral de formule générale XIII de configuration R ou S au niveau du carbone en position 2 : 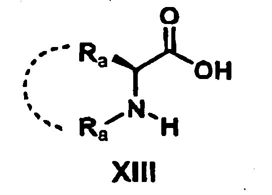 dans laquelle les deux substituants Rₐ, identiques ou différents, sont tels que définis ci-dessus, et peuvent former en outre un cycle, notamment de 5 à 8 éléments.

17. Procédé selon la revendication 16, **caractérisé en ce qu'**on réalise l'étape de condensation dans un solvant polaire ou ionique, en présence d'un catalyseur recyclable, ce qui conduit, selon la stéréochimie du catalyseur à l'un ou l'autre des composés de formule générale I, ou son image XIV : 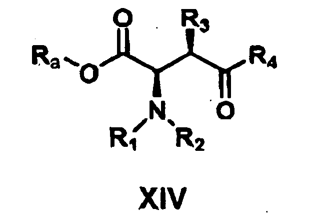 le composé XIV pouvant être soumis aux mêmes étapes que le composé de formule I.

18. Procédé selon la revendication 1, **caractérisé par** l'introduction d'un groupement R₂, différent d'un atome d'hydrogène, dans le composé de formule I.

19. Procédé selon la revendication 16, **caractérisé en ce que** les imines de formule XII sont obtenues par condensation d'un glyoxalate XV : 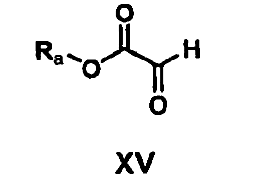 avec une amine R₁NH₂.

20. Procédé d'obtention de la 2S, 3R, 4S, 4-hydroxy-isoleucine de formule A selon la revendication 1, **caractérisé en ce qu'**il comprend :
- la condensation de la 2-butanone avec l'imine de formule XVI : 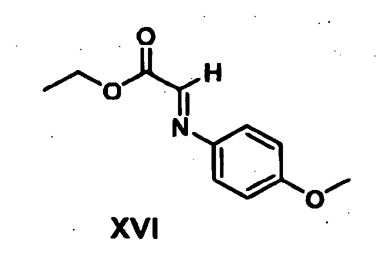
- en présence de L-proline, ce qui conduit, de manière majoritaire, au composé de formule XVII : 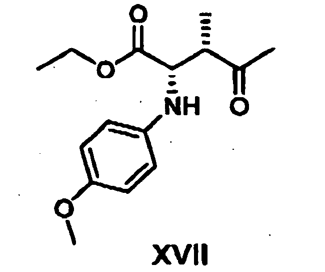
- suivi de son isomérisation/cristallisation par traitement de XVII avec de la DBU ou de la DBN, ce qui conduit au composé XVIII : 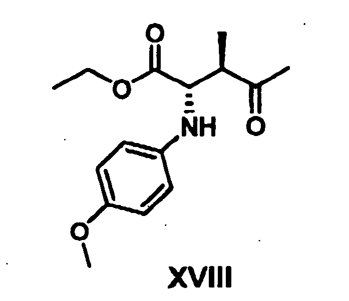
- l'élimination du groupement protecteur p-méthoxyphényle par traitement avec un persulfate,
- la réduction du composé déprotégé avec KBH₄/CeCl₃ dans de l'éthanol ou dans l'eau, ce qui conduit à la lactone XX: 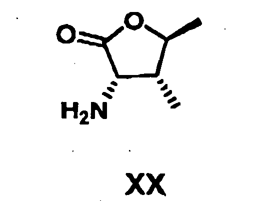
qui par hydrolyse dans LiOH/THF conduit majoritairement au composé A.

## Claims

1. A process for synthesizing, alone or in mixtures, the two isomers, at the OH function, of the α amino acids, or their derivatives, of the general formula B: 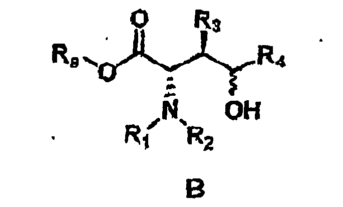 in which
- the C-O bond of the carbon in position 4 (represented by " 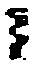 ") symbolizes one or other of the III or IV isomers, or their mixtures,
- R₁ and R₂ represent:
· a hydrogen atom, or
· either R₁ or R₂ represents a hydrogen atom and the other substituent is a radical Rₐ, an acyl group -CORₐ, or else a functional group -COORₐ, -SO₂Rₐ or -N (Rₐ,R_{b}), with Rₐ and R_{b}, which are identical or different, being a linear or branched C1-C12 alkyl radical, which is optionally substituted, an aryl group having one or more aromatic and heterocycle rings comprising 5 to 8C, which is optionally substituted, or aralkyl, with the alkyl substituent and the aryl group being as defined above, or
· R₁ and R₂ both represent a substituent as defined above,
- R₃ represents a hydrogen atom or Rₐ, and
- R₄ has the meanings of Rₐ,
**characterized in that** it comprises:
- the isomerization of a compound of the formula I: 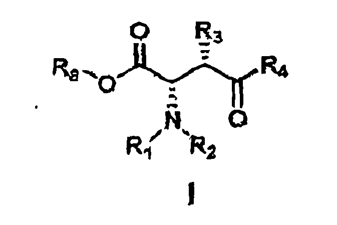
in which R₁, R₂, Rₐ, R₃ and R₄ are as defined above,
so as to obtain a compound of the formula II: 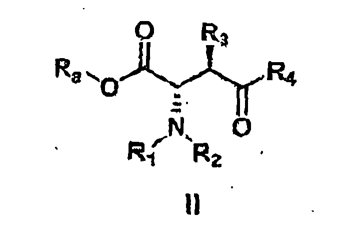
- the reduction of its carbonyl function, which leads, depending on the catalytic system which is used and the compound of the formula I which is employed, to one of the isomers of the general formula III or VI or to their mixture of the formula B: 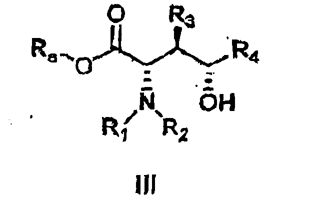 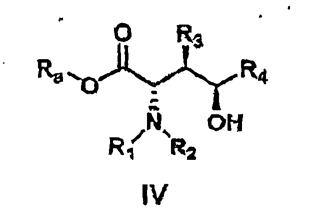
with the substituents Rₐ and R₁ to R₄ being as defined above.

2. The process as claimed in claim 1, **characterized in that** the isomerization step is followed by a step of total or partial deprotection of the amine function and then by a step of reduction of the carbonyl function so as to lead to, as the major or sole compound, the lactone of the formula VII 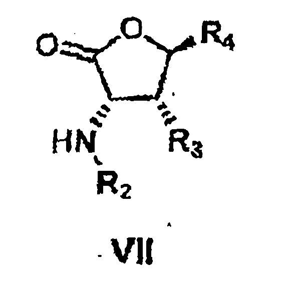 the opening of which leads to a mixture which mainly comprises the compound of the formula C

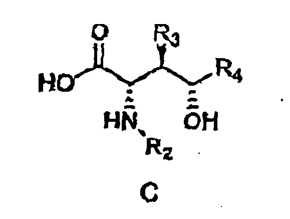

3. The process as claimed in claim 1, **characterized in that** the isomer III obtained by isomerization of the compound I and reduction of the compound II undergoes hydrolysis of its ester function in order to lead to the compound of the formula V: 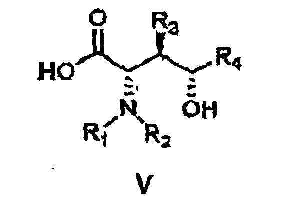 at least one of whose groups protecting the nitrogen function is eliminated, if desired, in order to lead to the compound of the general formula C:

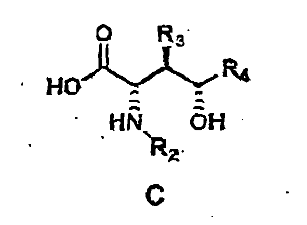

4. The process as claimed in claim 3, **characterized in that** the protective group R1 of the compound III is eliminated, with this leading to the compound of the formula VI: 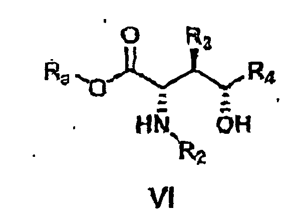 whose ester function is hydrolyzed in order to lead to the compound C.

5. The process as claimed in claim 3, **characterized in that** the compound of the formula VI is cyclized to form a lactone of the formula VII: 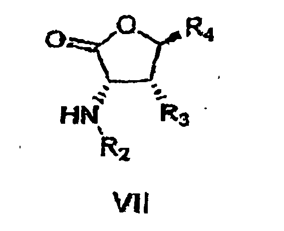 with the opening of this lactone then leading to the compound of the formula C, or **in that** the isomer II is reduced and then cyclized to form a compound of the formula VIII: 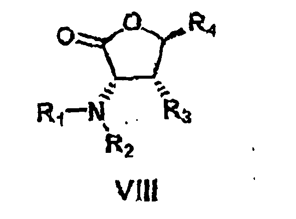 which is deprotected to form the compound of the formula VII, then leading to the compound of the formula C.

6. The process as claimed in claim 1, **characterized in that** the isomer of the formula IV is subjected to the sequences as claimed in claim 2 or 3, with this leading to a product of the general formula D:

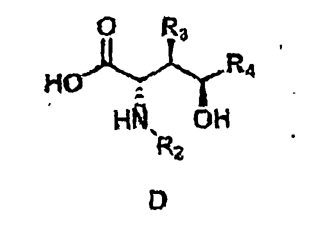

7. The process as claimed in claim 1, **characterized in that** the isomer II is reduced and cyclized so as to lead to a lactone of the formula VIII or IX: 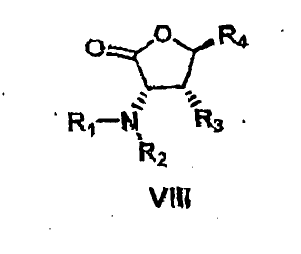 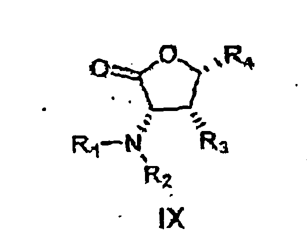 with it being possible to separate these lactones, if desired.

8. The process as claimed in claim 6, **characterized in that** the lactone VIII is converted into a lactone VII by total or partial deprotection of the amine function and the lactone IX is converted into a lactone X: 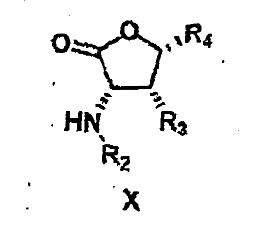 with these lactones VII and X then being hydrolyzed in order to lead to the compounds of the general formulae C and D according to claim 2.

9. The process as claimed in any one of claims 1 to 3, **characterized in that** the isomerization step is carried out with or without solvent, in the presence of a base, by creating conditions for precipitating the compound II, with this displacing the equilibrium towards this compound.

10. The process as claimed in any one of claims 1 to 3, **characterized in that** the isomer II is subjected to a step of total or partial deprotection of the amine function, with this step being carried out such that when R₁ represents an oxidizable protective group, recourse is had, to cerium and ammonium nitrate or to the electrochemical route, or use is made, as reagents, of persulfates, perborates, manganese dioxide, potassium permanganate, H₂O₂, FeCl₃ or a combination of cerium compound and an oxidizing agent.

11. The process as claimed in claim 10, **characterized in that** the persulfates are selected between those of ammonium, sodium, potassium.

12. The process as claimed in claim 10, **characterized in that** the deprotected product is subjected to a step of reduction, in aqueous medium, of the carbonyl function, with this predominantly leading to the compound VII.

13. The process as claimed in claim 1, **characterized in that** the isomer II which is recovered at the end of the isomerization step is subjected to a step of reduction of the carbonyl function.

14. The process as claimed in claim 13, **characterized in that** the hydrolysis of the ester function of the products III and IV is effected by treating in alkaline medium, or in an aqueous alcoholic solvent.

15. The process as claimed in any one of claims 4, 6 or 7, **characterized in that** the lactones of the formulae VIII or IX are hydrolyzed using LiOH in THF.

16. The process as claimed in any one of the preceding claims, **characterized in that** the compound of the formula I is obtained by condensing a ketone of the formula XI: 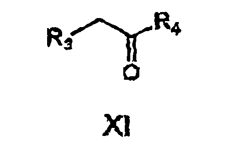 with an imine of the formula XII: 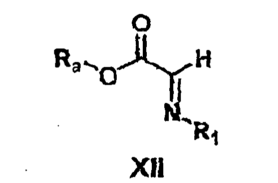 in the presence of a chiral catalyst of the general formula XIII, which can be of the R or S configuration at the carbon in position 2: 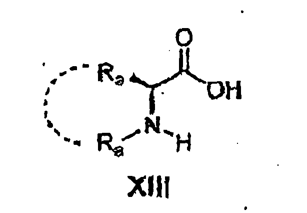 and in which the two substituents Rₐ, which are identical or different, are as defined above and can additionally form a cycle, in particular of from 5 to 8 members.

17. The process as claimed in claim 16, **characterized in that** the condensation step is carried out in a polar or ionic solvent and in the presence of a recyclable catalyst, with this leading, depending of the stereochemistry of the catalyst, to one or other of the compounds of the general formula I or to its mirror image XIV: 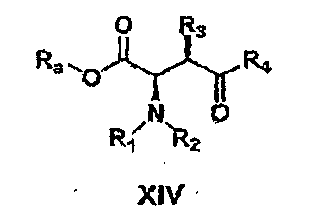 with it being possible for the compound XIV to be subjected to the same steps as the compound of the formula I.

18. The process as claimed in claim 1, **characterized by** the introduction of a group R₂, which is different from a hydrogen atom, into the compound of the formula I.

19. The process as claimed in claim 16, **characterized in that** the imines of the formula XII are obtained by condensing a glyoxalate XV: 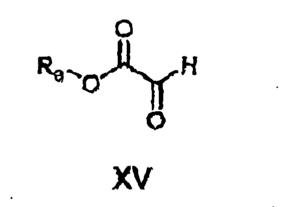 with an amine R₁NH₂.

20. A process for obtaining the 2S, 3R, 4S, 4-hydroxyisoleucine of the formula A as claimed in claim 1, **characterized in that** it comprises:
- the condensation of 2-butanone with the imine of the formula XVI: 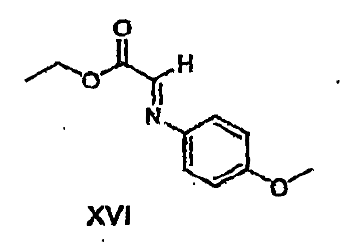
- in the presence of L-proline, which predominantly leads to the compound of the formula XVII: 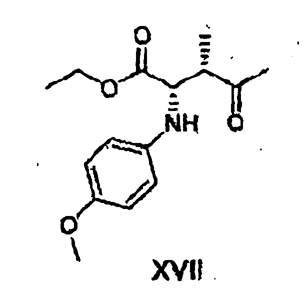
- followed by its isomerization/crystallization by means of treating XVII with DBU or DBN, which leads to the compound XVIII: 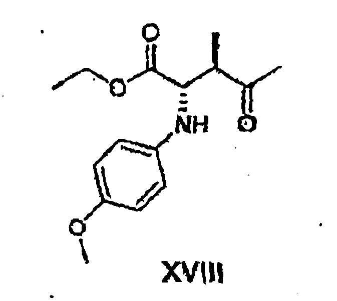
- elimination of the p-methoxyphenyl protective group by treating with a persulfate,
- reduction of the deprotected compound with KBH₄/CeCl₃ in ethanol or water, which leads to the lactone XX: 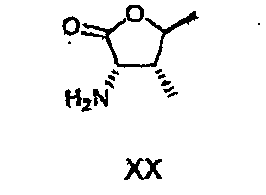
which, by means of hydrolysis in LiOH/THF, predominantly leads to the compound A.

## Patentansprüche

1. Verfahren zur Synthese der beiden bezüglich der OH-Funktion existierenden Isomere der α-Aminosäuren oder ihrer Derivate der allgemeinen Formel B 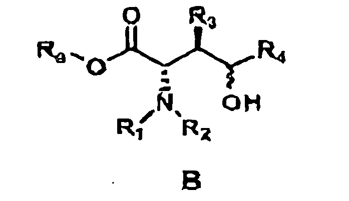 allein oder im Gemisch, wobei:
- die C-O-Bindung des Kohlenstoffs auf Position 4, die durch " 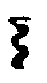 " dargestellt ist, für eines der beiden Isomere III oder IV oder ihre Gemische steht;
- R₁ und R₂ für:
• ein Wasserstoffatom stehen oder
• einer der Reste R₁ oder R₂ für ein Wasserstoffatom steht und der andere Substituent ein Rest Rₐ, eine Acylgruppe -CORₐ oder auch eine funktionelle Gruppe -COORₐ, -SO₂Rₐ, -N(Rₐ,R_{b}) ist, wobei Rₐ und R_{b} gleich oder verschieden sind und ein linearer oder verzweigter C₁-C₁₂-Alkylrest sind, der gegebenenfalls substituiert ist, eine Arylgruppe mit einem oder mehreren aromatischen und heterocyclischen Ringen mit 5 bis 8 C-Atomen, die gegebenenfalls substituiert ist, oder Aralkyl sind, wobei der Alkylsubstituent und die Arylgruppe wie oben definiert sind, oder
- R₁ und R₂ beide für einen Substituenten stehen, wie er oben definiert ist;
- R₃ für ein Wasserstoffatom oder Rₐ steht; und
- R₄ die Bedeutungen von Rₐ hat;
**dadurch gekennzeichnet, dass** es Folgendes umfasst:
- Isomrisierung einer Verbindung der Formel I: 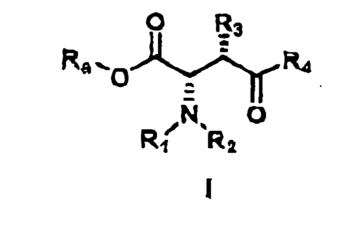
wobei R₁, R₂, Rₐ, R₃ und R₄ wie oben definiert sind;
wobei man eine Verbindung der Formel II erhält: 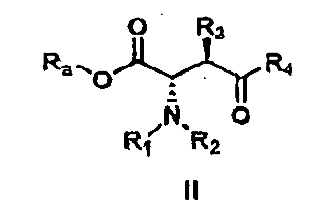
- Reduzieren der Carbonylfunktion, was je nach dem verwendeten Katalysatorsystem und der eingesetzten Verbindung der Formel I zu einem der Isomere der allgemeinen Formel III oder IV oder zu ihrem Gemisch der Formel B führt: 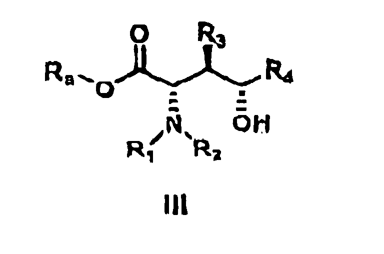 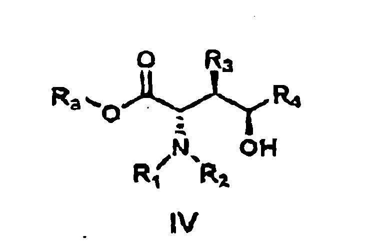
wobei die Substituenten Rₐ und R₁ bis R₄ wie oben definiert sind.

2. Verfahren gemäß Anspruch 1, **dadurch gekennzeichnet, dass** nach dem Schritt des Isomerisierens ein Schritt des vollständigen oder partiellen Entfernens der Schutzgruppen von der Aminfunktion und dann ein Schritt des Reduzierens der Carbonylfunktion durchgeführt werden, was als hauptsächliche oder ausschließliche Verbindung zu einem Lacton der Formel VII führt: 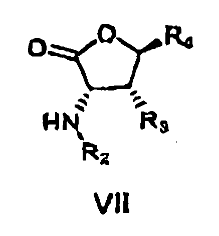 dessen Öffnung zu einem Gemisch führt, das hauptsächlich die Verbindung der Formel C umfasst:

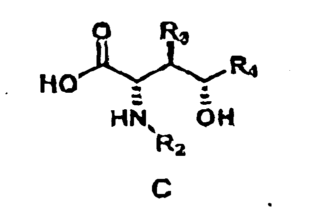

3. Verfahren gemäß Anspruch 1, **dadurch gekennzeichnet, dass** das durch Isomerisierung der Verbindung I und Reduktion der Verbindung II erhaltene Isomer III einer Hydrolyse seiner Esterfunktion unterzogen wird, was zur Verbindung der Formel V führt: 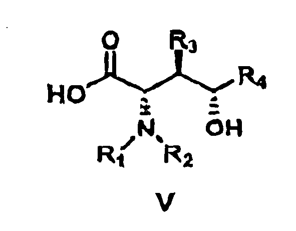 von der gegebenenfalls wenigstens eine der Schutzgruppen für die Stickstofffunktion entfernt wird, was zur Verbindung der allgemeinen Formel C führt:

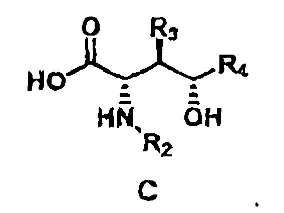

4. Verfahren gemäß Anspruch 3, **dadurch gekennzeichnet, dass** die Schutzgruppe R1 der Verbindung III entfernt wird, was zur Verbindung der Formel VI führt: 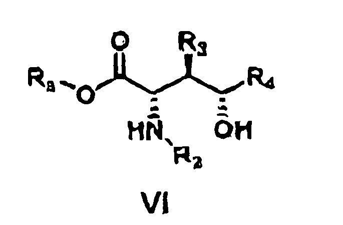 deren Esterfunktion hydrolysiert wird, was zur Verbindung C führt.

5. Verfahren gemäß Anspruch 3, **dadurch gekennzeichnet, dass** die Verbindung der Formel VI zu einem Lacton der Formel VII cyclisiert wird: 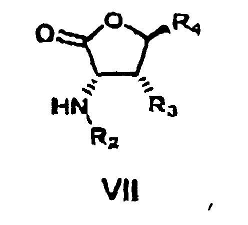 wobei die Öffnung dieses Lactons dann zur Verbindung der Formel C führt, oder dass das Isomer II reduziert und dann zu einer Verbindung der Formel VIII cyclisiert wird: 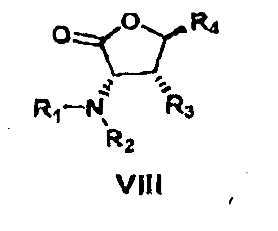 die von Schutzgruppen befreit wird, wobei man die Verbindung der Formel VII erhält, die anschließend zur Verbindung der Formel C führt.

6. Verfahren gemäß Anspruch 1, **dadurch gekennzeichnet, dass** das Isomer der Formel IV den Reaktionsfolgen gemäß den Ansprüchen 2 oder 3 unterzogen wird, was zu einem Produkt der allgemeinen Formel D führt:

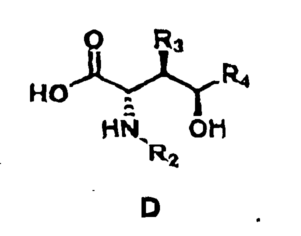

7. Verfahren gemäß Anspruch 1, **dadurch gekennzeichnet, dass** das Isomer II reduziert und cyclisiert wird, was zu einem Lacton der Formel VIII oder IX führt: 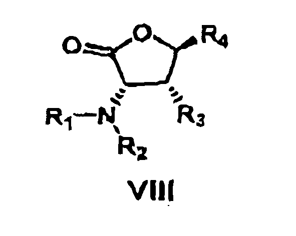 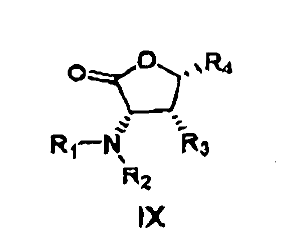 wobei diese Lactone gegebenenfalls voneinander getrennt werden können.

8. Verfahren gemäß Anspruch 6, **dadurch gekennzeichnet, dass** das Lacton VIII durch vollständiges oder partielles Entfernen der Schutzgruppen von der Aminfunktion zum Lacton VII und das Lacton IX zum Lacton X umgewandelt wird: 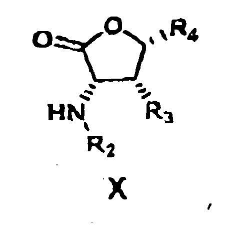 wobei diese Lactone VII und X anschließend hydrolysiert werden, was zu den Verbindungen der allgemeinen Formeln C bzw. D gemäß Anspruch 2 führt.

9. Verfahren gemäß einem der Ansprüche 1 bis 3, **dadurch gekennzeichnet, dass** der Schritt des Isomerisierens mit oder ohne Lösungsmittel In Gegenwart einer Base durchgeführt wird, indem man Bedingungen herstellt, unter denen die Verbindung II ausgefällt wird, was das Gleichgewicht zu dieser Verbindung hin verschiebt.

10. Verfahren gemäß einem der Ansprüche 1 bis 3, **dadurch gekennzeichnet, dass** das Isomer II einem Schritt des vollständigen oder partiellen Entfernens der Schutzgruppen von der Aminfunktion unterzogen wird, wobei dieser Schritt in dem Fall durchgeführt wird, dass R₁ für eine oxidierbare Schutzgruppe steht, dass man Ammoniumcernitrat verwendet oder auf elektrochemischem Weg oxidiert oder dass man als Reagentien Persulfate, Perborate, Mangandioxid, Kaliumpermanganat, H₂O₂, FeCl₃ oder die Kombinationen von Cer und Oxldationsmittel verwendet.

11. Verfahren gemäß Anspruch 11, **dadurch gekennzeichnet, dass** die Persulfate aus Ammonium-, Natrium- oder Kaliumpersulfat ausgewählt werden.

12. Verfahren gemäß Anspruch 10, **dadurch gekennzeichnet, dass** das von Schutzgruppen befreite Produkt einem Schritt der Reduktion der Carbonylfunktion in wässrigem Medium unterzogen wird, was vorwiegend zur Verbindung VII führt.

13. Verfahren gemäß Anspruch 1, **dadurch gekennzeichnet, dass** das am Ende des Isomerisierungsschritts gewonnene Isomer II einem Schritt der Reduktion der Carbonylfunktion unterzogen wird.

14. Verfahren gemäß Anspruch 13, **dadurch gekennzeichnet, dass** die Hydrolyse der Esterfunktion der Produkte III oder IV durch Behandlung in alkalischem Medium oder in einem wässrigen alkoholischen Medium erfolgt.

15. Verfahren gemäß einem der Ansprüche 4, 6 oder 7, **dadurch gekennzeichnet, dass** die Lactone der Formeln VIII oder IX hydrolysiert, wobei man LiOH in THF verwendet.

16. Verfahren gemäß einem der vorstehenden Ansprüche, **dadurch gekennzeichnet, dass** die Verbindung der Formel I durch Kondensation eines Ketons der Formel XI 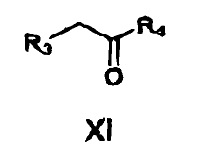 mit einem Imin der Formel XII 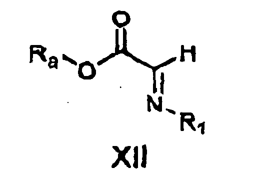 in Gegenwart eines chiralen Katalysators der allgemeinen Formel XIII mit R- oder S-Konfiguration in Bezug auf den Kohlenstoff auf Position 2 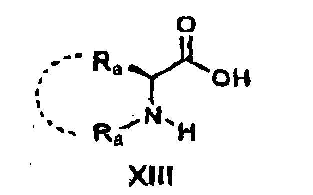 erhalten wird, wobei die beiden Substituenten Rₐ gleich oder verschieden und wie oben definiert sind und außerdem einen Ring, insbesondere mit 5 bis 8 Gliedern, bilden können.

17. Verfahren gemäß Anspruch 16, **dadurch gekennzeichnet, dass** der Kondensationsschritt in einem polaren oder ionischen Lösungsmittel in Gegenwart eines wiederverwendbaren Katalysators durchgeführt wird, was je nach der Stereochemie des Katalysators zu einem der beiden Verbindungen der allgemeinen Formel I oder ihrem Spiegelbild XIV führt: 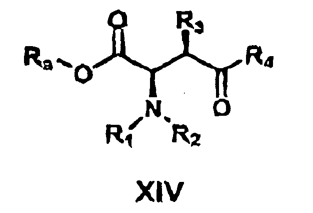 wobei die Verbindung XIV denselben Schritten wie die Verbindung der Formel I unterzogen werden kann.

18. Verfahren gemäß Anspruch 1, **dadurch gekennzeichnet, dass** eine Gruppe R₂, die von einem Wasserstoffatom verschieden ist, in die Verbindung der Formel I eingeführt wird.

19. Verfahren gemäß Anspruch 16, **dadurch gekennzeichnet, dass** die Imine der Formel XII durch Kondensation eines Glyoxalats XV 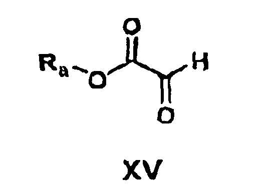 mit einem Amin R₁NH₂ erhalten werden.

20. Verfahren zur Gewinnung von 2S,3R,4S-4-Hydroxyisoleucin der Formel A gemäß Anspruch 1, **dadurch gekennzeichnet, dass** es Folgendes umfasst:
- die Kondensation von 2-Butanon mit dem Imin der Formel XVI: 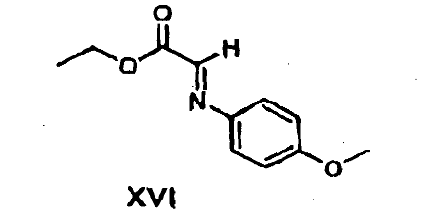
- in Gegenwart von L-Prolin, was vorwiegend zur Verbindung der Formel XVII führt 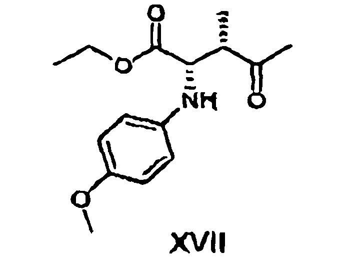
- gefolgt von deren Isomerisierung/Kristallisation durch Behandlung von XVII· mit DBU oder DBN, was zur Verbindung XVIII führt: 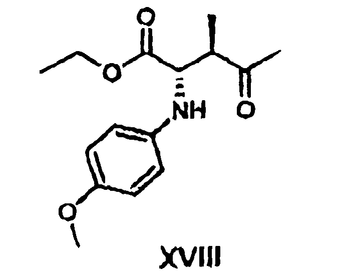
- Beseitigung der p-Methoxyphenyl-Schutzgruppe durch Behandlung mit einem Persulfat;
- Reduktion der von Schutzgruppen befreiten Verbindung mit KBH₄/CeCl₃ in Ethanol oder in Wasser, was zum Lacton XX führt: 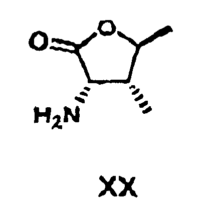
das zur Hydrolyse in LiOH/THF vorwiegend zur Verbindung A führt.
